# EUROPEAN PATENT APPLICATION

(11) **EP 3 964 123 A1**
(43) Date of publication of application: **09.03.2022**
(21) Application number: 21195158.7
(22) Date of filing: 06.09.2021
(51) Int. Cl.: A61B 5/00

(54) **A BRAIN-COMPUTER INTERFACE**

(30) Priority: 06.09.2020 US 202063075192 P
(71) Applicant: Cerebrolytics Inc, Chicago, IL 60610 (US)
(72) Inventor: Siemionow, Kris, Chicago (US); Paul, Lewicki, Tulsa (US)
(74) Representative: Kancelaria Eupatent.pl Sp. z.o.o

(57) **Abstract**

A communicating device for placing within a blood vessel, the communicating device comprising: a proximal end part (10); a terminal end part (20); a wired connector (30) that communicatively connects the proximal end part (10) with the terminal end part (20); wherein the proximal end part (10) comprises a battery (101) connected to a wireless charging terminal (102) and a signal processing circuit (103) connected to a wireless communication terminal (104); and wherein the terminal end part (20) comprises a plurality of sensors (201), electrodes (202) and microfilaments (23, 203) configured to be deployed through the walls of the vessel (50).

## Description

### TECHNICAL FIELD

This disclosure is related to the field of medical devices, in particular to a brain-computer interface.

### BACKGROUND

Many people suffer from pharmacologically resistant depression, epilepsy, addiction. Moreover, to date there is no cure for neurodegenerative disorders such as Alzheimer's or Parkinson's. State of the art understanding of brain disorders is limited to observational and postmortem analysis. Currently, obtaining real-time data from a diseased brain is very limited.

A PCT application WO2017070252 discloses devices, methods, and systems for transmitting signals through a device located in a blood vessel of an animal, for stimulating and/or sensing activity of media proximal to the device, wherein the media includes tissue and/or fluid. The device comprises a frame structure forming a plurality of struts, where the frame structure is moveable between a reduce profile and an expanded profile in which a diameter of the frame structure increases. At least one of the plurality of struts forming the frame structure comprises an electrically conductive material on a support material, the electrically conductive material extending along at least a portion of the strut and being covered with a non-conductive material. At least one electrode is formed by an opening in the non-conductive material on the portion of the strut. A lead is located at an end of the frame structure and configured to be in electrical communication with the electrically conductive portion, the lead extending from the frame structure.

### SUMMARY

There is a need to further develop the area of brain-computer interface to overcome several technical disadvantages of state of the art.

In one aspect, the invention relates to a communicating device for placing within a blood vessel, the communicating device comprising: a proximal end part; a terminal end part; a wired connector that communicatively connects the proximal end part with the terminal end part; wherein the proximal end part comprises a battery connected to a wireless charging terminal and a signal processing circuit connected to a wireless communication terminal; and wherein the terminal end part comprises a plurality of sensors, electrodes and microfilaments configured to be deployed through the walls of the vessel.

The proximal end part may have a diameter greater than the diameter of the terminal end part.

The proximal end part can be configured to be placed in a jugular vein.

The proximal end part can be configured to be placed in a vein in an arm or a leg.

The proximal end part can be connected to an antenna.

The terminal end part may comprise a collapsible and expandable stent for affixing the terminal end part at a desired location and a sleeve that houses the sensors, the electrodes and the microfilaments.

The proximal end part may comprise a collapsible and expandable stent for affixing the proximal end part at a desired location and a sleeve that houses the battery, the wireless charging terminal, the signal processing circuit and the wireless communication terminal.

The terminal end part may comprise a micro-robotic device configured to deploy microfilaments through the walls of the vessel and into the surrounding neural structures.

The microfilaments may have a form of microneedles.

The microfilaments can be housed in an expandable and collapsible balloon inserted into the stent.

The microfilaments can be spring loaded and protected by a sheath.

The microfilaments may have a form of barbs on hooks outside the stent.

The microfilaments can be configured to detect, to stimulate or to deliver substances.

The device has various applications. For example, the terminal end can be positioned in superficial cortical veins to stimulate motor cortex (in case of functional deficits of a patient) or in internal jugular vein to stimulate anterior nucleus (in case of a patient suffering from epilepsy, Alzheimer's). Furthermore, the terminal end can be positioned in the anterior communicating artery to interact with nucleus acumbens, subgenual cingulate white matter (depression), ventral capsule (obsessive compulsive disorder, addiction, depression).

These and other features, aspects and advantages of the invention will become better understood with reference to the following drawings, descriptions, and claims.

### BRIEF DESCRIPTION OF DRAWINGS

Various embodiments are herein described, by way of example only, with reference to the accompanying drawings, wherein:
Fig. 1 shows schematically an embodiment for a communicating device for placing within a blood vessel;
Fig. 2 shows the device of Fig. 1 placed within the blood vessel;
Fig. 3 shows a more detailed schematic view of a terminal end part of the device of Fig. 1;
Fig. 4 shows positioning of a proximal end part of the device of Fig. 1;
Fig. 5 shows a functional schematic of the device;

### DETAILED DESCRIPTION OF THE EMBODIMENTS

The following detailed description is of the best currently contemplated modes of carrying out the invention. The description is not to be taken in a limiting sense but is made merely for the purpose of illustrating the general principles of the invention.

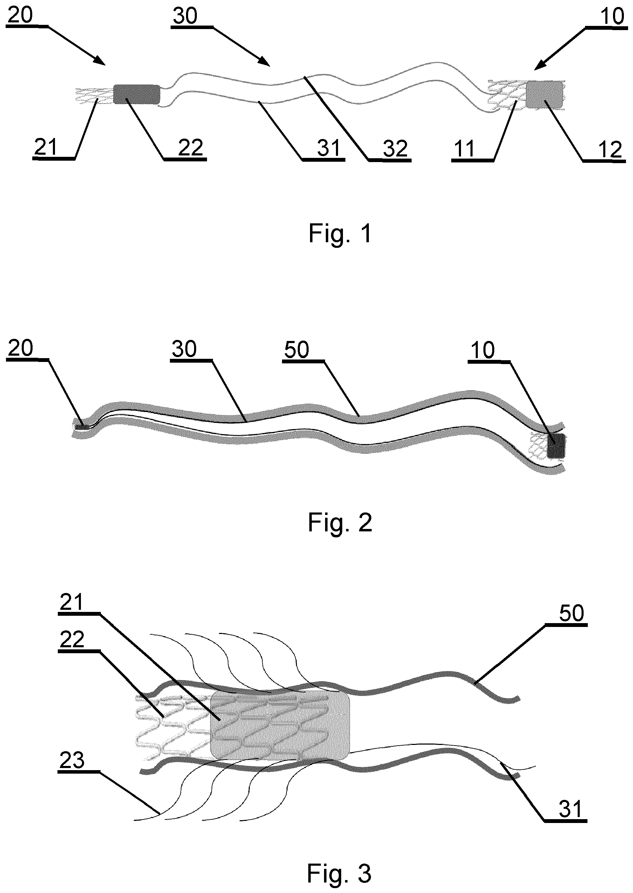

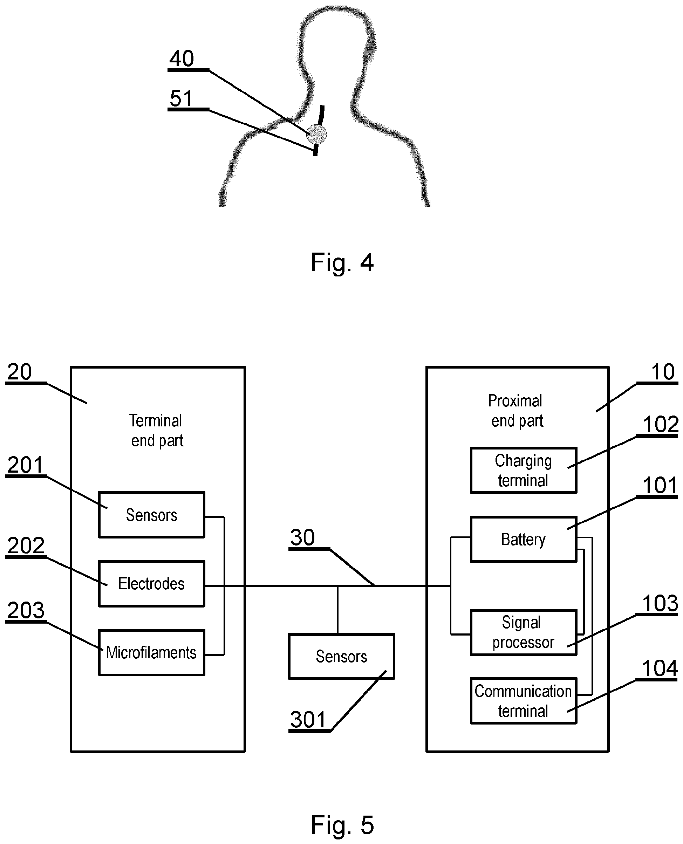

The communicating device for placing within a blood vessel has a structure as shown in an example in Fig. 1 and functional elements as shown in an example in Fig. 5. The device comprises a proximal end part 10, a terminal end part 20 and a wired connector 30 that connects the proximal end part 10 with the terminal end part 20.

The device is supposed to be placed within a blood vessel, in particular within one of intracranial blood vessels, such as internal cerebral vein, great cerebral vein, basal veins, inferior anastomotic vein, superficial middle cerebral vein, superior anastomotic vein, thalamostriate veins, cortical vein, ophthalmic vein.

The device can be placed within the blood vessel by an introducer device, such as a steerable guidewire capable of traversing complex vasculature anatomy, that deploys the terminal end part 20 at a desired location within the intracranial blood vessels system and deploys the proximal end part 10 at a jugular vein 51 as shown in Fig. 4. Other locations for the terminal end part 20 are possible as well - preferably, these are locations that are close to the skin surface to allow for communication and charging, such as veins in an arm or leg close to skin surface that are particularly preferable because these locations can be covered by smart clothes.

The terminal end part 20 is configured to sense and/or stimulate the anatomy parts at its vicinity, to provide stimulation, navigation (e.g., infrared and EM based) and/or guidance (e.g., AI based). It comprises a collapsible and expandable stent 21 and a sleeve 22. The stent 21 has a form of a net that can adjust its diameter so that it becomes adjacent to the internal walls of the blood vessel wherein the terminal end part 20 is positioned, so that it functions as an anchor for affixing the terminal end part 20 at a desired location. The sleeve 22 has a cylindrical shape, for example of a diameter of about 3 mm and a length of about 10 mm. The sleeve 22 houses various sensors 201 and electrodes 202 to sense and/or stimulate the target area. In particular, the sleeve 22 houses a micro-robotic device (i.e., an endovascular robot) that, when coupled to the introducer, deploys microfilaments 23, 203 through the walls of the vessel 50 and into the surrounding neural structures. For example, this can be done by expanding a balloon inserted into the center of the stent that deploys microneedles, and next collapsing and removing the balloon. Alternatively, the microneedles can be spring loaded and protected by a sheath, and when the sheath is removed or dissolved with time (e.g., the sheath can be made from polyglycolic acid or some other biocompatible material), the needles spring out from the stent, pierce the vessel and enter the brain matter. Furthermore, the needles can have a form of barbs on hooks outside the stent, while the stent can be advanced past its ideal position and then pulled back which makes the microneedles catch the tissue and then get pushed into the vessel wall, and through it, as the stent is pulled back. The microfilaments may have a form of microneedles, such as described in a publication "Microneedles: A smart approach and increasing potential for transdermal drug delivery system" by Waghule, Tejashree, et al. in Biomedicine & pharmacotherapy 109 (2019): 1249-1258. The microfilaments interact by picking up and delivering electrical signals. Therefore, some of the microfilaments can be electroconductive. Therefore, some of the microfilaments can be used to detect the brain waves or brain activity, while some of the microfilaments can be used to deliver neurotransmitters like dopamine or serotonin. The microfilaments 23, 203 allow to interface with a large cerebral surface area.

The proximal end part 10 comprises a collapsible and expandable stent 11 and a sleeve 12. The stent 11 has a form of a net that can adjust its diameter so that it becomes adjacent to the internal walls of the blood vessel wherein the proximal end part 10 is positioned, so that it functions as an anchor for affixing the proximal end part 10 at a desired location. The sleeve 12 has a cylindrical shape, for example of a diameter of about 10 mm and a length of about 20 mm. The sleeve 12 houses a battery 101, a charging terminal 102, signal processing circuits 103 and signal transmitting terminal 104.The signal processing circuit 103 collects data from the sensors 201 and process them e.g., to form data packets for transmission. Further, the signal processing circuit 103 reads incoming data to correspondingly control the electrodes 202. The charging terminal 102 and the transmitting terminal 104 may be of a wireless type and they can be connected to an antenna 40 that is located in a vicinity of the proximal end part 10. The antenna 40 allows communication with an external charging and signal transmission circuitry. Positioning of the proximal end part 10 in the jugular vein makes the proximal end part 10 and the antenna 40 easily accessible, as it is close to the skin surface.

A single proximal end part 10 may be communicatively coupled with a plurality of terminal end parts 20, each terminal end part 20 located at a different location within the brain.

The wired connector 30 has a form of wires 31 that transmit power and signals between the proximal end part 10 and the terminal end part 20. The wired connector 30 may further comprise various sensors 301 so as to sense the vessel in a region between the locations of the end parts 10, 20.

The sensors 201, 301 used in the terminal end part 20 and/or the wired connector 30 may be of various types. Non-limiting examples of sensors include sensors for measurement of dopamine, N-acetylaspartate (NAA), creatine, glutamate, choline, lactate, glutamate, myo-inositol, homovanilic acid, glucose, cholesterol, aminoacids: alanine, valine, leucine and isoleucine, ghrelin, leptin, electrolytes: Na+, K+, Ca++, Mg++.

Particulars of the components of the device that have not been elaborated in detail in this description are known to skilled persons and do not require to be further explained. A skilled person will realize e.g., what type of stent and sensors to use for the terminal end part, how to connect by wire the terminal end part with the proximal end part or how to wirelessly charge the battery and transmit data.

While the invention has been described with respect to a limited number of embodiments, it will be appreciated that many variations, modifications, and other applications of the invention may be made. Therefore, the claimed invention as recited in the claims that follow is not limited to the embodiments described herein.

## Claims

1. A communicating device for placing within a blood vessel, the communicating device comprising:
- a proximal end part (10);
- a terminal end part (20);
- a wired connector (30) that communicatively connects the proximal end part (10) with the terminal end part (20);
- wherein the proximal end part (10) comprises a battery (101) connected to a wireless charging terminal (102) and a signal processing circuit (103) connected to a wireless communication terminal (104);
- and wherein the terminal end part (20) comprises a plurality of sensors (201), electrodes (202) and microfilaments (23, 203) configured to be deployed through the walls of the vessel (50).

2. The communicating device according to claim 1, wherein the proximal end part (10) has a diameter greater than the diameter of the terminal end part (20).

3. The communicating device according to any of previous claims, wherein the proximal end part (10) is configured to be placed in a jugular vein.

4. The communicating device according to any of claims 1-2, wherein the proximal end part (10) is configured to be placed in a vein in an arm or a leg.

5. The communicating device according to any of previous claims, wherein the proximal end part (10) is connected to an antenna (40).

6. The communicating device according to any of previous claims, wherein the terminal end part (20) comprises a collapsible and expandable stent (21) for affixing the terminal end part (20) at a desired location and a sleeve (22) that houses the sensors (201), the electrodes (202) and the microfilaments (203).

7. The communicating device according to any of previous claims, wherein the proximal end part (10) comprises a collapsible and expandable stent (11) for affixing the proximal end part (10) at a desired location and a sleeve (12) that houses the battery (101), the wireless charging terminal (102), the signal processing circuit (103) and the wireless communication terminal (104).

8. The communicating device according to any of previous claims, wherein the terminal end part (20) comprises a micro-robotic device configured to deploy microfilaments (23, 203) through the walls of the vessel (50) and into the surrounding neural structures.

9. The communicating device according to any of previous claims, wherein the microfilaments (23, 203) have a form of microneedles.

10. The communicating device according to any of previous claims, wherein the microfilaments (23, 203) are housed in an expandable and collapsible balloon inserted into the stent.

11. The communicating device according to any of previous claims, wherein the microfilaments (23, 203) are spring loaded and protected by a sheath.

12. The communicating device according to any of previous claims, wherein the microfilaments (23, 203) have a form of barbs on hooks outside the stent.

13. The communicating device according to any of previous claims, wherein the microfilaments (23, 203) are configured to detect, to stimulate or to deliver substances.
